# FASCICULE DE BREVET EUROPEEN

(11) **EP 1 890 760 B1**
(45) Date de publication et mention de la délivrance du brevet: **11.06.2008**
(21) Numéro de dépôt: 06842085.0
(22) Date de dépôt: 04.12.2006
(51) Int. Cl.: A61M 39/10

(54) **CONNECTEUR À USAGE MÉDICAL**
STECKVERBINDER ZUR MEDIZINISCHEN VERWENDUNG
CONNECTOR FOR MEDICAL USE

(30) Priorité: 05.12.2005 FR 0553720
(43) Date de publication de la demande: 27.02.2008
(73) Titulaire: CAIR LGL, 69380 Civrieux d'Azergues (FR)
(72) Inventeur: LOPEZ, Georges-Antoine, F-69290 Craponne (FR); DELORME, Patrick, F-69630 Chaponost (FR); ALLARD, Ludovic, F-69390 Millery (FR)
(74) Mandataire: Denjean, Eric
(86) Numéro de dépôt international: PCT/FR2006/051274
(87) Numéro de publication internationale: WO 2007/066034

(56) Documents cités:
- EP-B- 0 681 493
- EP-B- 0 781 151
- US-A1- 2004 217 315
- US-A1- 2005 151 105

## Description

L'invention a pour objet un connecteur à usage médical. Le connecteur dont il est question est du type présentant une première extrémité également désignée "extrémité amont" destinée à être connectée à une tubulure dans laquelle circule un fluide ; et une seconde extrémité, désignée également "extrémité aval" destiné à coopérer avec un dispositif de prélèvement ou d'injection dudit fluide par le biais d'un connecteur du type luer mâle. Dans la suite de la description, on se réfère aux expressions « extrémité amont » et « extrémité aval » quel que soit le sens de circulation du fluide.

En pratique, le dispositif présente de manière connue, une chambre solidaire à l'une de ses extrémités d'un raccord constituant l'extrémité amont du connecteur proprement dit, l'autre extrémité de la chambre étant conformée pour recevoir, par friction, l'embout du connecteur du type luer mâle. Le passage du liquide entre la tubulure connectée à l'extrémité amont du connecteur et l'extrémité du luer mâle est assuré par le biais d'une aiguille solidaire du raccord et débouchant dans la chambre.

Des connecteurs de ce type ont largement été décrits, l'une des difficultés essentielles étant d'assurer une étanchéité satisfaisante de la connexion avec l'embout de la seringue.

Les dispositifs supposés répondre à cet objectif sont par exemple ceux décrits dans le document EP-A-148 635. Ce document décrit en effet un connecteur à clapet muni d'une tête de piston élastiquement déformable maintenue dans une position directement adjacente au raccord aval de jonction, le piston délimitant lui même une chambre à l'intérieur de laquelle est agencée l'aiguille. En position non activée du connecteur, la tête de piston est dans son état déformé, provoquant ainsi l'obturation du perçage et donc la fermeture de la chambre. Au contraire, en position activée, la tête de piston est dans son état non déformée, l'orifice de sortie de la tête de piston restant alors ouvert pouvant ainsi être traversée par l'aiguille. En d'autres termes, une quantité de liquide correspondant sensiblement au volume de la chambre reste en permanence dans ladite chambre, le risque étant qu'une partie s'écoule par le perçage sous l'effet de la pression.

Le document EP-A-681 493 décrit une valve médicale munie d'un joint élastique, là encore adjacent à la paroi de l'extrémité libre du connecteur et destiné à être comprimé sous l'effet de la poussée exercée par le dispositif d'injection ou de prélèvement de fluide.

Dans un mode de réalisation particulier et à la différence du précédent document, le joint recouvrant l'aiguille est en contact avec celle-ci sur toute sa hauteur permettant d'interdire la formation de volume résiduel en position non activée. La compression du joint élastique permet dès lors de découvrir l'extrémité de l'aiguille et ainsi de libérer ses orifices pour permettre le transfert du fluide dans la seringue.

Le document EP-A-544 581 décrit un connecteur comportant un ressort muni en son centre d'une aiguille surmontée par un bouchon en élastomère, ledit bouchon étant susceptible de glisser dans le corps du connecteur entre une position de repos et une position activée. En pratique, le bouchon est traversé par ladite aiguille en position activée du système, alors qu'il est obturé en position de repos, le ressort constituant le moyen élastique entraînant le déplacement du bouchon.

Le principal inconvénient de ces différents dispositifs, dont le principe commun est d'agencer un ressort élastique directement au contact des parois de la chambre, est de ne pas garantir une étanchéité optimale, du fait notamment du serrage radial trop limité lié à la nature même du matériau élastique.

Le document EP-A-309 771 décrit un connecteur muni non pas d'une aiguille mais d'une canule présentant une extrémité plate, gainée par un joint élastique, le joint étant surmonté au niveau de son extrémité aval, par une douille de serrage en matière plastique. Il ressort des figures qu'une partie du joint élastique reste en contact avec les parois de la chambre, interdisant ainsi, du fait de la force de friction élevée, un guidage homogène et régulier dudit joint élastique et donc un transfert de fluide constant. En outre et surtout, eu égard à la force de serrage radiale appliquée à l'extrémité du joint élastique, on peut s'attendre, compte tenu de l'agencement de la douille, du joint, et de la canule, à une résistance importante du joint au moment de l'introduction de l'extrémité de la seringue et donc à un passage difficile de la canule au travers de la fente terminale.

Le document EP 781 151 B1 décrit un connecteur apte à recevoir un luer mâle. Le connecteur est muni d'une gaine enveloppant l'aiguille, la gaine, malgré la présence d'un moyen de serrage, étant directement au contact de la paroi interne de l'extrémité terminale du connecteur.

Le Demandeur a cherché à améliorer les systèmes décrits dans ces documents avec pour principaux objectifs d'assurer une étanchéité optimale du connecteur et un transfert constant de fluide en position activée.

Pour ce faire, le Demandeur a perfectionné le connecteur à bague tel que précédemment décrit en prévoyant, dans la zone séparant l'extrémité de l'aiguille de l'extrémité du joint élastique, un évidement du joint et ou de la bague permettant ainsi de refouler la matière au moment du passage de l'aiguille, sans pour autant mettre en péril l'étanchéité du système.

En d'autres termes, l'invention a pour objet un connecteur à usage médical comprenant un raccord muni en son centre d'une aiguille s'étendant dans une chambre et débouchant éventuellement dans partie de l'extrémité terminale de ladite chambre, ladite extrémité terminale présentant une section apte à recevoir, par friction, un connecteur type luer mâle, l'aiguille étant gainée et maintenue, au moins dans sa partie terminale incluant l'orifice, dans l'empreinte d'un joint élastique présentant, dans l'épaisseur de son extrémité libre, une fente ou équivalent, le joint élastique ayant une extrémité libre tangente à celle de la chambre et étant muni d'une bague cerclant sa partie terminale au moins jusque dans la zone en regard du ou des orifices de l'aiguille.

Ce connecteur se caractérise en ce que sur partie de la longueur de l'extrémité terminale de la chambre, la surface externe du joint élastique et/ou la surface interne ou l'épaisseur de la bague présente au moins un évidement destiné à favoriser le refoulement de la matière constitutive du joint, au moment du passage de l'aiguille.

En pratique, la bague s'étend, en amont du ou des orifices de l'aiguille, sur une longueur inférieure à 3 mm, en pratique de l'ordre de 2 mm.

Selon l'invention, l'évidement peut donc être formé sur deux pièces distinctes. Ainsi, l'évidement peut être formé uniquement sur la surface externe du joint élastique. Il peut être également formé uniquement sur la surface interne de la bague ou dans l'épaisseur de la bague. Dans ce dernier cas, il s'agit alors d'un ajourement. Il peut enfin être formé dans les deux éléments. Dans cette hypothèse, l'évidement prévu dans le joint élastique est avantageusement en regard de l'évidement prévu dans la bague, de sorte à optimiser le phénomène de refoulement de matière.

Le nombre d'évidement n'est pas limité.

Dans un mode de réalisation préféré, la surface externe du joint élastique présente un évidement annulaire tandis que la bague présente, dans son épaisseur, deux évidements répartis symétriquement.

Le ou les orifices de l'aiguille peuvent être prévus en plusieurs endroits.

Dans une première forme de réalisation, l'aiguille présente une extrémité conique, plane ou arrondie, et est munie d'un orifice terminal.

Pour assurer le passage sans contrainte de l'aiguille dans la fente, le ou les évidements sont formés dans une zone en aval de l'extrémité de l'aiguille.

Pour assurer l'étanchéité du système, c'est-à-dire le serrage du joint élastique entraînant la fermeture de la fente, la section externe du joint en contact avec la bague en aval de l'évidement, est supérieure à la section interne correspondante de la bague. En revanche, en amont du ou des évidements, la section externe du joint est sensiblement égale à la section interne de la bague.

Par ailleurs, pour assurer l'étanchéité entre l'aiguille et le joint élastique en position non connectée du connecteur, l'aiguille est de section supérieure à la section interne correspondante du joint, c'est-à-dire de la section de l'empreinte, sur toute la partie du joint cerclée par la bague. En amont de cette zone, l'aiguille est de section sensiblement égale ou inférieure à la section interne correspondante du joint.

De même, pour favoriser le perçage de la fente par l'aiguille, l'empreinte du joint élastique dans laquelle est maintenue l'aiguille peut être plus longue que l'aiguille, en pratique de quelques millimètres (au maximum 2 mm).

Dans une autre forme de réalisation, l'aiguille présente une extrémité conique, plane ou arrondie et est munie d'au moins un, avantageusement de deux orifices latéraux en regard l'un de l'autre.

Pour assurer le passage sans contrainte de l'aiguille dans la fente, le ou les évidements sont formés dans une zone en aval de ladite extrémité de l'aiguille.

Dans ce mode de réalisation, l'étanchéité n'est pas assurée au niveau de la fente mais au niveau des orifices latéraux.

Dans ce cas, la section externe du joint, à l'exclusion de l'évidement, est sensiblement égale à la section interne de la bague et ce, sur toute la longueur de la bague.

En revanche et de même que précédemment, l'aiguille est de section supérieure à la section interne correspondante du joint, sur toute la partie du joint cerclée par la bague. En amont de cette zone, l'aiguille est de section sensiblement égale ou inférieure à la section interne correspondante du joint.

Dans la mesure où les orifices sont perpendiculaires par rapport au flux de liquide, et que l'aiguille est soumise dans cette zone à la contrainte du joint élastique, il n'y a aucune possibilité de reflux de liquide. L'étanchéité est donc optimale.

Le corps de l'aiguille peut adopter différentes formes telles que par exemple une forme tubulaire, conique épaulée ou non par palier, l'extrémité pouvant être plane, pointue (conique) ou arrondie.

Selon l'invention, le joint peut recouvrir toute ou partie de l'aiguille. Dans un mode de réalisation avantageux, il recouvre la totalité de la longueur de l'aiguille.

Lorsque le joint ne recouvre pas toute l'aiguille, ledit joint est rendu solidaire de tout moyen élastique et notamment un ressort, venant prendre appui sur la base du raccord.

Pour en améliorer la souplesse et ainsi favoriser le dégagement de l'aiguille, ledit joint élastique présente, dans sa partie médiane, au moins une, avantageusement deux fentes latérales.

Dans un autre mode de réalisation, l'extrémité amont et la partie médiane du joint, présentent une forme générale conique, la paroi du joint dans cette zone se présentant sous la forme d'une succession de bourrelets ou boudins.

De même, pour assurer le guidage du joint dans la chambre, ledit joint élastique n'a aucun point de contact avec les parois latérales de la chambre. Dès lors, le guidage est assuré par la seule bague, dont tout ou partie de la paroi est en contact glissant avec la paroi correspondante de la chambre pendant toute la durée du mouvement du joint élastique, de sa position de repos à sa position comprimée. Du fait de la nature plastique des matériaux utilisés, il existe un minimum seulement de force de friction entre la chambre et la bague.

Le joint élastique est en effet fabriqué avantageusement en matériau élastomère, tel que par exemple silicone ou thermoplastique, et plus généralement en tout matériau susceptible de lui conférer une élasticité suffisante pour permettre le dégagement de l'aiguille tout en assurant, en position de repos, l'obturation étanche, suivant les modes de réalisation, au niveau de la fente ou au niveau des orifices latéraux.

En pratique, la bague est fabriquée en un matériau rigide ou semi-rigide.

Dans une première forme de réalisation, elle est fabriquée séparément pour ensuite être associée au joint élastique avant mise en place dans le connecteur.

Dans une seconde forme de réalisation, l'ensemble joint élastique-bague est bi matière, c'est-à-dire fabriqué au sein d'un seul moule au moyen de deux matières distinctes.

Dans une configuration préférée, la chambre présente au moins deux compartiments de section distincte, respectivement un compartiment central de section interne constante, au moins dans la zone correspondant à la course de la bague, et un compartiment terminal constituant l'extrémité terminale, de section interne inférieure constante ou non, la bague s'étendant en partie, dans le compartiment central en position de repos du connecteur.

En pratique la section interne du compartiment terminal peut être cylindrique ou du type luer femelle, c'est-à-dire présentant un cône luer à 6%. Pour favoriser le guidage du joint élastique, la partie de la bague contenue dans le compartiment central est de forme cylindrique. En revanche, dans le compartiment central, la bague peut avoir une forme distincte qui sera fonction de la forme de la section interne dudit compartiment.

L'invention et les avantages qui en découlent ressortiront bien des exemples de réalisation suivants, à l'appui des figures annexées.
La figure 1 est une représentation en perspective du connecteur de l'invention couvrant deux modes de réalisation possible en fonction de la nature de l'aiguille soit, à orifice terminal, soit à orifices latéraux.
La figure 2 est une vue en coupe de la figure 1, lorsque l'aiguille présente deux orifices latéraux et dans laquelle le joint élastique est en position précontrainte.
La figure 3 est une représentation en perspective de l'ensemble raccord-aiguille de la figure 2.
La figure 4 est une représentation en perspective de l'ensemble joint élastique-bague.
Les figures 5 et 6 sont des vues en coupe de la figure 4.
La figure 7 est une vue en coupe de la chambre.
La figure 8 est une représentation en coupe du connecteur en position comprimée du joint élastique, c'est-à-dire après connexion du connecteur du type luer mâle. Dans ce mode de réalisation, l'aiguille présente des orifices latéraux.
La figure 9 est un second mode de réalisation dans lequel l'orifice de l'aiguille est terminal.
La figure 10 est une représentation en coupe du connecteur de l'invention, dans lequel le joint élastique présente une succession de bourrelets.
La figure 11 est une représentation du joint élastique de la figure 10 en perspective.

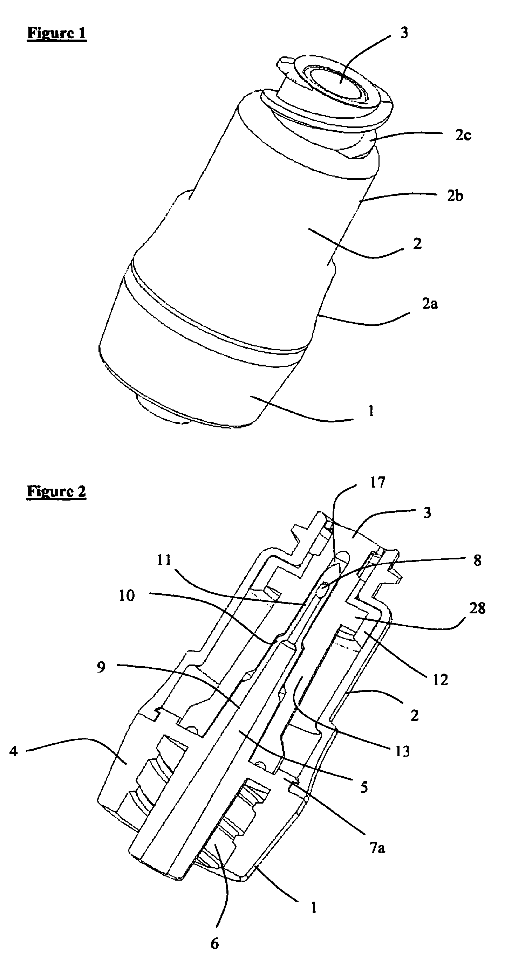

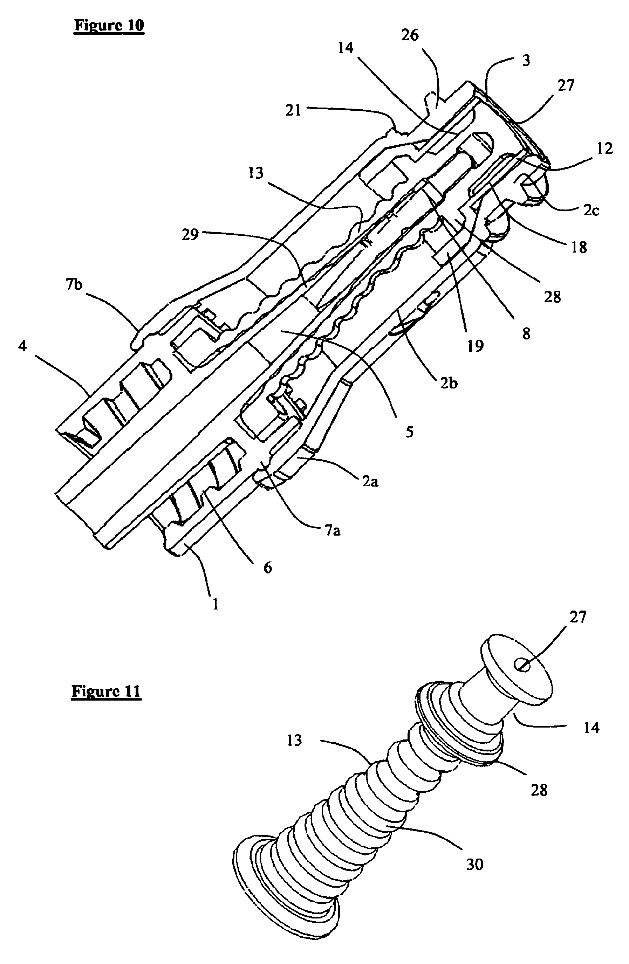

La figure 1.2 est une coupe de la figure 11.

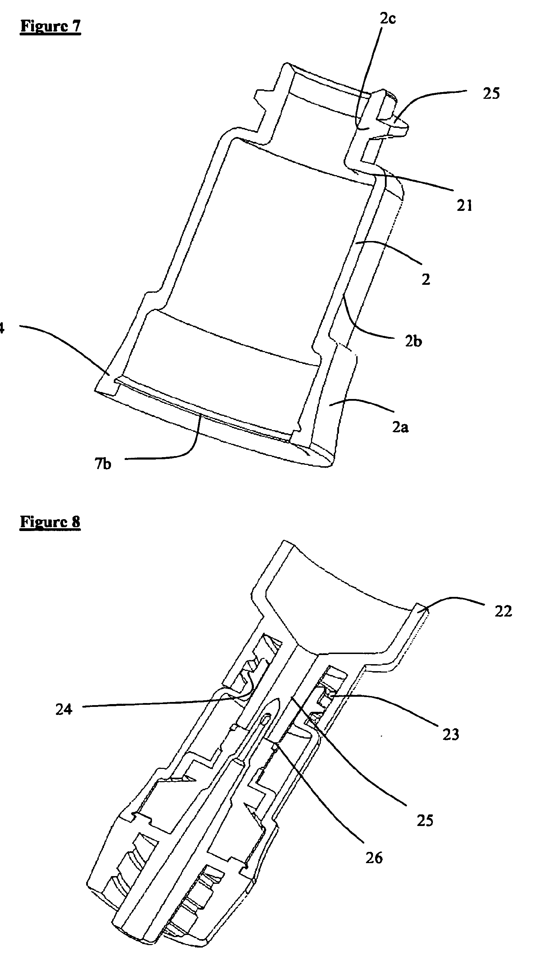

On a représenté sur la figure 1, une vue en perspective du connecteur de l'invention. Ce connecteur est constitué de trois pièces distinctes, respectivement un raccord (1), une chambre (2) comprenant un premier compartiment (2a), un compartiment central (2b) et un compartiment terminal (2c) ainsi qu'un ensemble bague-joint élastique (3) (voir aussi figure 7).

On retrouve ces trois éléments associés, illustrés sur la figure 2, en coupe, en position de repos du joint élastique dans le mode de réalisation dans lequel l'aiguille présente deux orifices latéraux.

Plus précisément, le raccord (1) se présente sous la forme d'une pièce composite associant le corps du raccord proprement dit (4) et l'aiguille (5). Le corps du raccord est en outre muni d'un pas de vis intérieur (6) destiné à coopérer avec un pas de vis correspondant d'un connecteur luer femelle dans laquelle peut circuler un fluide. Ce raccord est en outre muni d'un clip (7a) destiné à coopérer cette fois, avec une forme correspondante (7b), agencée à la base de la première chambre (2a). L'aiguille (5) proprement dite est munie, à proximité de son extrémité libre, elle-même obturée, de deux orifices latéraux (8) par lesquels s'écoule le fluide. Cette aiguille présente en outre, de sa base jusqu'à son extrémité libre, trois tronçons (9, 10, 11) de section décroissante conique séparés par des épaulements, destinés à favoriser son dégagement sous l'effet de la poussée exercée par le connecteur type luer mâle sur l'ensemble bague/joint élastique (3).

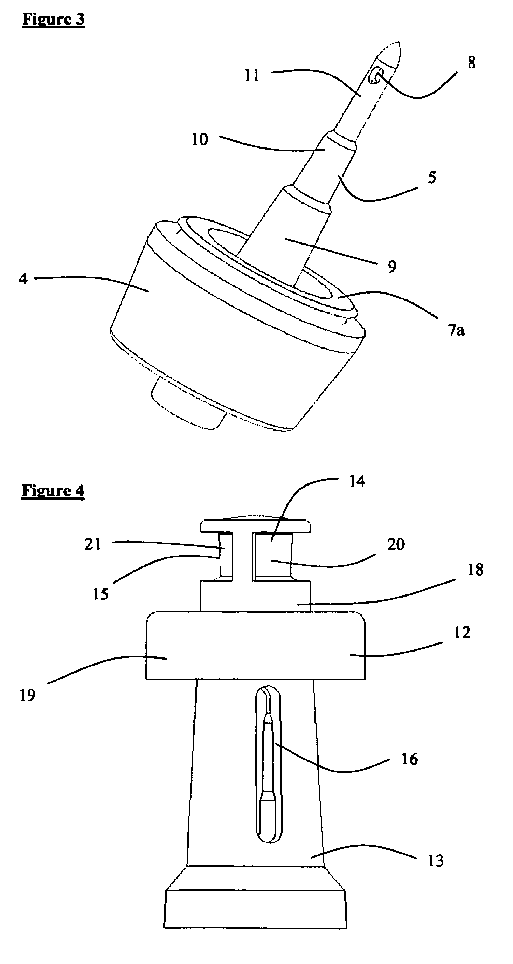

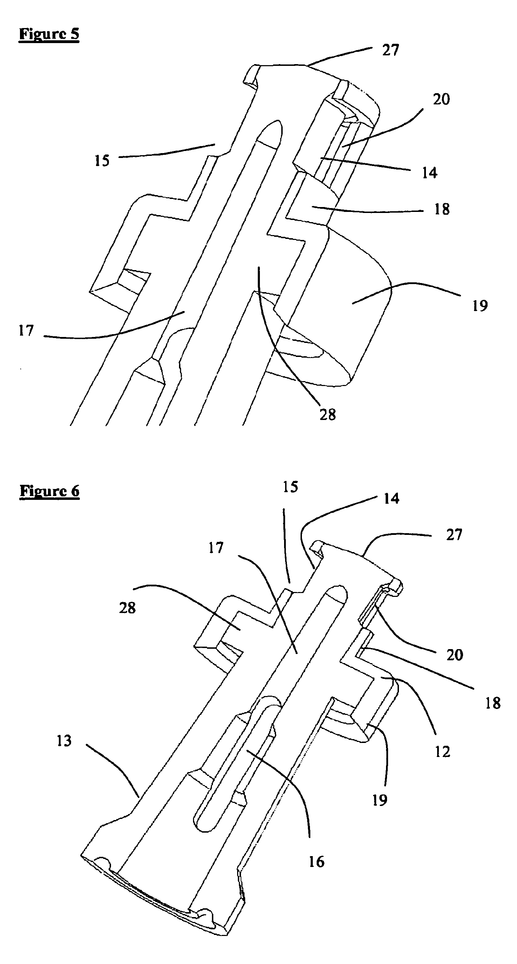

Sur les figures 4, 5 et 6, est représenté le corps de l'invention, à savoir l'ensemble bague (12)-joint élastique (13). Plus précisément, le joint élastique se présente sous la forme d'un tube de section externe conique, destiné à s'étendre de la chambre (2a) depuis la base du raccord (1) jusqu'à l'extrémité libre de la chambre terminale (2c). Le joint élastique n'est en aucun point en contact avec la chambre (en position non comprimée). Pour tenir compte de la forme et des dimensions de la chambre centrale en combinaison avec la fonction de guidage de la bague, le joint présente à proximité de l'extrémité aval de la chambre centrale (2b), une collerette (28) cerclée par la bague (12). Ce joint élastique est réalisé en silicone et présente, à proximité de son extrémité libre, un évidement (14), ainsi qu'une fente (27). Comme il apparaît sur la figure 2, l'évidement est en amont de l'extrémité de l'aiguille en position non comprimée du joint élastique. Pour améliorer sa souplesse et favoriser son déplacement à la fois sous l'effet de la poussée exercée sur la bague, mais également au moment de son repositionnement, le joint élastique est muni de deux fentes latérales (16), en regard l'une de l'autre, agencées sur la partie médiane. En outre, comme le montrent les figures 5 et 6, le joint élastique présente en son centre, une empreinte (17) de forme correspondante à celle de l'aiguille, l'empreinte étant toutefois légèrement plus longue que l'aiguille.

Dans le mode de réalisation représenté sur la figure 2, la section interne du joint élastique est supérieure à la section de l'aiguille mise à part dans la zone terminale de l'aiguille recouverte par la bague et incluant les orifices latéraux, au niveau de laquelle la section interne du joint élastique est inférieure à la section de l'aiguille.

La bague (12) est notamment illustrée sur les figures 4 et 5. Elle est positionnée à l'extrémité du joint élastique (13) et présente deux tronçons de sections différentes, respectivement un premier tronçon cylindrique (18) cerclant la partie terminale du joint élastique sur une longueur correspondant à la longueur du compartiment terminal et de section sensiblement égale à la section dudit compartiment et un second tronçon cylindrique (19), de section supérieure sensiblement égale à la section correspondante de la chambre centrale (2b) et recouvrant la collerette (28) du joint élastique. La bague (12) est réalisée en un matériau rigide ou semi-rigide, éventuellement en bi-matière avec le joint élastique, ou séparément, bague et joint étant rendus solidaires l'un de l'autre notamment par collage ou simple apposition. Dans le mode de réalisation dans lequel les trous de l'aiguille sont prévus latéralement (figure 2), la section externe du joint est sensiblement égale à la section interne de la bague sur leur zone de recouvrement.

Comme le montrent les figures 4, 5, 6, la partie terminale (18) de la bague (12) présente deux ajourements (15, 20) destinés à favoriser le refoulement de la matière plastique au moment du passage de l'aiguille sous l'effet de la poussée créée par la mise en place du connecteur type luer mâle.

Comme illustré sur la figure 7, le compartiment central (2b) et le compartiment terminal (2c) sont reliés par un épaulement (21) servant de butée au joint élastique en position de repos garantissant ainsi que l'extrémité du joint élastique soit tangente à l'extrémité de la chambre terminale (en position de repos).

La figure 2 représente l'agencement de ces différentes pièces entre-elles, en position de repos de l'ensemble joint élastique-bague. Plus précisément, dans une telle position, la totalité de la surface externe de la bague est en contact glissant avec la surface interne correspondante des chambres centrale et terminale du connecteur. Dans cette configuration, la partie (19) de la bague de section plus importante vient comme déjà dit, en butée contre l'épaulement correspondant (21) du connecteur. Dans cette même position, le joint élastique n'a aucun point de contact avec les faces latérales de la chambre. Par ailleurs, du fait du choix des sections des différents éléments, aucune force radiale n'est exercée par la bague sur le joint élastique (sections sensiblement égales), la fermeture des orifices étant assurée par la force radiale du joint élastique exercée sur l'aiguille (section du joint inférieure à section de l'aiguille).

La position connectée est plus précisément représentée sur la figure 8 par la mise en place d'un connecteur type luer mâle (22). Ce connecteur présente un pas de vis (23) coopérant avec le pas de vis correspondant (24) de la chambre terminale (2c), permettant ainsi d'assurer une fixation efficace du dispositif d'injection ou de prélèvement de fluide via le connecteur luer. Le connecteur est en outre muni d'un cône luer (25), destiné à être insérée dans la lumière de la chambre terminale (2c). En pratique, le cône (25) vient prendre appui par son extrémité (26) sur l'extrémité libre de l'ensemble bague-joint élastique. Cet appui provoque le déplacement du joint élastique le long du fût de l'aiguille en direction du raccord, puis le passage de la pointe de l'aiguille au travers de la fente (27) facilité par les évidements, et enfin la libération des orifices latéraux et partant le passage du fluide.

En position connectée, la partie libre de l'aiguille est donc dans sa totalité, contenue dans le canal intérieur du cône (25), permettant ainsi la transmission du fluide de connecteur à connecteur. Le mouvement de la bague pendant cette opération, est un mouvement axial homogène et uniforme de par le contact permanent des parois de la section (19) de la bague avec la chambre centrale (2b) tout au long du mouvement, moyennant un minimum de friction. Comme le montre la figure 8, en position compressée du joint élastique, la partie de la bague de faible section est en contact ni avec la chambre terminale, ni avec la chambre centrale. En revanche, le joint élastique vient dans ce cas, en contact avec les parois latérales de la chambre. En pratique, le volume de la chambre est prévu pour pouvoir contenir le volume du joint en position comprimée.

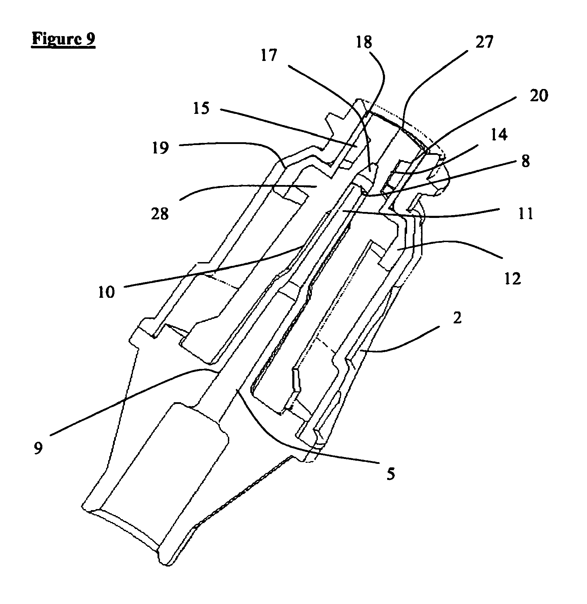

Sur la figure 9, on a représenté le mode de réalisation dans lequel l'orifice de l'aiguille est terminal. Dans le cas d'espèce, l'extrémité libre de l'aiguille est une extrémité plane. Pour favoriser l'insertion de cette aiguille dans la fente, l'empreinte a une longueur légèrement plus élevée que celle de l'aiguille (2 mm de plus). A la différence du mode de réalisation à deux orifices latéraux, l'étanchéité est assurée non pas au niveau de l'orifice de l'aiguille, mais au niveau de la fente. Pour obtenir cette étanchéité, une force radiale est exercée par la bague (12) sur l'extrémité du joint élastique, la partie (18) de la bague ayant une section interne inférieure à la section externe du joint élastique. En revanche, la section externe du joint élastique au niveau de la collerette (28) est sensiblement égale à la section interne de la partie (19) de la bague, dans la mesure où l'étanchéité n'est pas recherchée à ce niveau. Parallèlement, la section interne du joint, sur la longueur de l'aiguille en amont de la bague, est supérieure à la section de ladite aiguille. Sur la partie en regard de la bague, la section interne du joint élastique est inférieure à la section de l'aiguille.

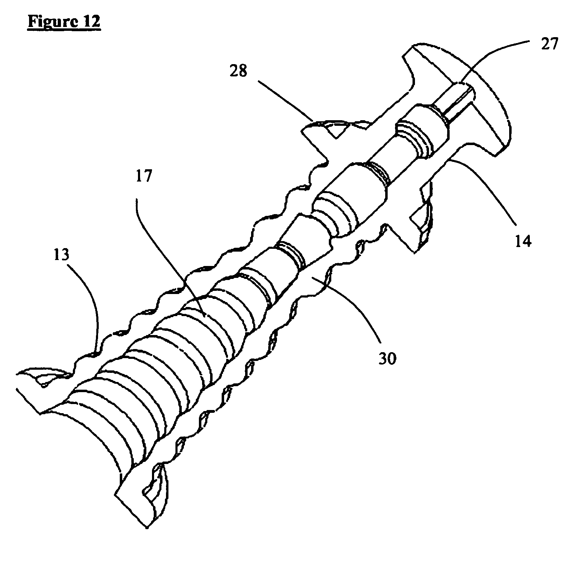

Les figures 10 à 12 représentent un autre mode de réalisation du connecteur de l'invention qui se distingue du mode de réalisation précédent en essentiellement trois points, respectivement :
- la forme de l'aiguille,
- la forme de la bague,
- la forme du joint élastique.

S'agissant tout d'abord de l'aiguille (5), celle-ci présente une section conique (29) de section décroissante de la base du connecteur jusqu'à son extrémité libre. A la différence du mode de réalisation précédent, il n'y a pas ici de paliers, du fait que le dégagement de l'aiguille, sous l'effet de la poussée exercée par le connecteur type luer mâle, est facilité par la forme même du joint élastique (voir ensuite).

S'agissant ensuite de la bague (12), celle-ci présente toujours deux tronçons de sections différentes, respectivement :
- un premier tronçon cylindrique (18) cerclant la partie terminale du joint élastique sur une longueur correspondant à la longueur du compartiment terminal et de section sensiblement égale à la section dudit compartiment,
- et un second tronçon cylindrique (19) de section sensiblement égale à la section correspondante de la chambre centrale (2b).

A la différence du mode de réalisation précédent, la bague dans son tronçon (18) n'est pas ajourée, mais présente au contraire une paroi continue dépourvue de tout évidement.

S'agissant enfin du joint élastique, celui-ci présente, dans sa partie terminale, une forme identique à celle décrite et faisant l'objet des figures 1 à 9, à savoir, dans la direction de son extrémité terminale, une collerette (28), un évidement (14) et une fente (27). En revanche, ce joint élastique se distingue de celui précédemment décrit dans la forme de la partie médiane et de l'extrémité amont, c'est-à-dire dans la forme en amont de la collerette (28). Celle-ci présente en effet une configuration conique, la paroi du joint étant munie de bourrelets (30) successifs. Cette forme spécifique confère au joint élastique une souplesse améliorée, favorisant son déplacement à la fois sous l'effet de la poussée exercée sur la bague, mais également au moment de son repositionnement.

Les avantages de l'invention ressortent bien de la description qui précède. On note en particulier l'étanchéité totale du système obtenue en amont de la fente terminale du connecteur.

## Revendications

1. Connecteur à usage médical comprenant un raccord (1) muni en son centre d'une aiguille (5) s'étendant dans une chambre (2) et débouchant éventuellement dans partie de l'extrémité terminale de ladite chambre, ladite extrémité terminale présentant une section apte à recevoir, par friction, un connecteur type luer mâle, l'aiguille (5) étant gainée au moins dans sa partie terminale incluant le ou les orifices (8) dans l'empreinte (17) d'un joint élastique (13) présentant, dans l'épaisseur de son extrémité libre, une fente (27) ou équivalent, le joint élastique (13) ayant une extrémité libre tangente à celle de la chambre (2) et étant muni d'une bague (12) cerclant sa partie terminale au moins jusque dans la zone en regard du ou des orifices (8) de l'aiguille, **caractérisé en ce que** sur partie de la longueur de l'extrémité terminale de la chambre (2), la surface externe du joint élastique (13) et/ou la surface interne ou l'épaisseur de la bague (12) présente au moins un évidement destiné à favoriser le refoulement de la matière constitutive du joint (13), au moment du passage de l'aiguille (5).

2. Connecteur selon la revendication 1, **caractérisé en ce que** l'évidement est ménagé sur la surface externe du joint élastique (13) et dans l'épaisseur de la bague (12).

3. Connecteur selon l'une des revendications précédentes, **caractérisé en ce que** la surface externe du joint élastique présente un évidement annulaire (14) tandis que la bague présente, dans son épaisseur, deux évidements répartis symétriquement (15,20).

4. Connecteur selon la revendication 1, **caractérisé en ce que** l'évidement est ménagé exclusivement sur la surface externe du joint élastique (13).

5. Connecteur selon l'une des revendications 1 à 4, **caractérisé en ce que** l'aiguille (5) présente une extrémité conique, plane ou arrondie et est munie d'un orifice terminal.

6. Connecteur selon la revendication 5, **caractérisé en ce que** la section externe du joint (13) en contact avec la bague (12) en aval de l'évidement, est supérieure à la section interne correspondante de la bague (12).

7. Connecteur selon l'une des revendications 5 à 6, **caractérisé en ce qu'**en amont du ou des évidements (14, 15, 20), la section externe du joint (13) est sensiblement égale à la section interne de la bague (12).

8. Connecteur selon l'une des revendications 1 à 4, **caractérisé en ce que** l'aiguille (5) présente une extrémité conique, plane ou arrondie et est munie de deux orifices latéraux (8) en regard l'un de l'autre.

9. Connecteur selon la revendication 8, **caractérisé en ce que** sur toute la longueur de la bague (12), la section externe du joint (13), à l'exclusion de l'évidement, est sensiblement égale à la section interne de la bague (12).

10. Connecteur selon l'une des revendications 5 à 9, **caractérisé en ce que** le ou les évidements (14, 15, 20) sont formés dans une zone en aval de l'extrémité de l'aiguille.

11. Connecteur selon l'une des revendications 5 à 10, **caractérisé en ce que** :
- l'aiguille (5) est de section supérieure à la section interne correspondante du joint (13), sur toute la partie du joint élastique cerclée par la bague (19).
- l'aiguille (5) est de section sensiblement égale ou inférieure à la section interne correspondante du joint (13) en amont de ladite partie.

12. Connecteur selon l'une des revendications précédentes, **caractérisé en ce que** le joint élastique (13) recouvre la totalité de la longueur de l'aiguille (5) et présente deux fentes latérales (16) ménagées dans sa partie médiane.

13. Connecteur selon l'une des revendications 1 à 11, **caractérisé en ce que** le joint élastique (13) recouvre la totalité de la longueur de l'aiguille (5) et a une forme conique, et **en ce que** la paroi présente, au niveau de ses parties médiane et de son extrémité amont, une succession de bourrelets.

14. Connecteur selon l'une des revendications précédentes, **caractérisé en ce que** le joint élastique (13) n'a aucun point de contact avec les parois latérales de la chambre (2) en position non comprimée.

15. Connecteur selon l'une des revendications précédentes, **caractérisé en ce que** tout ou partie de la paroi de la bague (19) est en contact glissant avec la paroi correspondante de la chambre (2) pendant toute la durée du mouvement du joint élastique (13) de sa position de repos à sa position comprimée.

16. Connecteur selon l'une des revendications précédentes, **caractérisé en ce que** la chambre (2) présente au moins deux compartiments de section distincte, respectivement un compartiment central (2b) de section!interne constante, au moins dans la zone correspondant à la course de la bague (12), et un compartiment terminal (2c) constituant l'extrémité terminale, de section interne inférieure et constante ou non, la bague (12) s'étendant en partie dans le compartiment central (2b).

17. Connecteur selon la revendication 16, **caractérisé en ce que** la section interne du compartiment terminal présente un cône luer à 6% et **en ce que** la partie de ls bague contenue dans le compartiment terminal est de forme cylindrique.

## Claims

1. Connector for medical use comprising an adapter (1) equipped at its centre with a needle (5) extending into a chamber (2) and optionally terminating in part of the terminal end of the said chamber, the said terminal end having a cross section suitable for receiving, by friction, a luer type male connector, the needle (5) being encased at least in its terminal part including the orifice or orifices (8) in the cavity (17) of an elastic seal (13) having, in the thickness of its free end, a slit (27) or equivalent, the elastic seal (13) having a free end at a tangent to that of the chamber (2) and being provided with a ring (12) encircling its terminal part at least as far as the zone opposite the orifice or orifices (8) of the needle, **characterized in that** along part of the length of the terminal end of the chamber (2), the outer surface of the elastic seal (13) and/or the inner surface or thickness of the ring (12) has at least one recess for promoting the expulsion of the material constituting the seal (13), at the time of passage of the needle (5).

2. Connector according to Claim 1, **characterized in that** the recess is made on the outer surface of the elastic seal (13) and in the thickness of the ring (12).

3. Connector according to either of the preceding claims, **characterized in that** the outer surface of the elastic seal has an annular recess (14) while the ring, in its thickness, has two symmetrically distributed recesses (15, 20).

4. Connector according to Claim 1, **characterized in that** the recess is made exclusively on the outer surface of the elastic seal (13).

5. Connector according to one of Claims 1 to 4, **characterized in that** the needle (5) has a conical, plane or rounded end and is provided with a terminal orifice.

6. Connector according to Claim 5, **characterized in that** the outer cross section of the seal (13) in contact with the ring (12) downstream of the recess, is larger than the corresponding inner cross section of the ring (12).

7. Connector according to either of Claims 5 and 6, **characterized in that** upstream of the recess or recesses (14, 15, 20), the outer cross section of the seal (13) is substantially equal to the inner cross section of the ring (12).

8. Connector according to one of Claims 1 to 4, **characterized in that** the needle (5) has a conical, plane or rounded end and is provided with two side orifices (8) facing one another.

9. Connector according to Claim 8, **characterized in that** along the whole length of the ring (12), the outer cross section of the seal (13), to the exclusion of the recess, is substantially equal to the inner cross section of the ring (12).

10. Connector according to one of Claims 5 to 9, **characterized in that** the recess or recesses (14, 15, 20) are formed in a zone downstream of the end of the needle.

11. Connector according to one of Claims 5 to 10, **characterized in that**:
- the needle (5) has a cross section larger than the corresponding inner cross section of the seal (13), along the whole part of the elastic seal encircled by the ring (19),
- the needle (5) has a cross section substantially equal to or lower than the corresponding inner cross section of the seal (13) upstream of the said part.

12. Connector according to one of the preceding claims, **characterized in that** the elastic seal (13) covers the entire length of the needle (5) and has two side slits (16) made in its mid-portion.

13. Connector according to one of Claims 1 to 11, **characterized in that** the elastic seal (13) covers the entire length of the needle (5) and has a conical shape, and **in that** the wall, in its mid-portions and at its upstream end, has a succession of ripples.

14. Connector according one of the preceding claims, **characterized in that** the elastic seal (13) has no contact point with the side walls of the chamber (2) in the uncompressed position.

15. Connector according to one of the preceding claims, **characterized in that** all or part of the wall of the ring (19) is in sliding contact with the corresponding wall of the chamber (2) throughout the movement of the elastic seal (13) from its rest position to its compressed position.

16. Connector according to one of the preceding claims, **characterized in that** the chamber (2) has at least two compartments with different cross sections, respectively a central compartment (2b) having a constant inner cross section, at least in the zone corresponding to the stroke of the ring (12), and a terminal compartment (2c) constituting the terminal end, having a lower and constant or variable inner cross section, the ring (12) extending partly into the central compartment (2b).

17. Connector according to claim 16, **characterized in that** the inner cross section of the terminal compartment has a 6% luer taper and **in that** the part of the ring contained in the terminal compartment has a cylindrical shape.

## Patentansprüche

1. Verbinder zum medizinischen Gebrauch, mit einem Anschlussstück (1), das in seiner Mitte mit einer Nadel (5) ausgestattet ist, die sich in einer Kammer (2) erstreckt und gegebenenfalls in einen Teil des abschließenden Endes der Kammer (2) mündet, wobei das abschließende Ende einen Abschnitt aufweist, der dazu ausgelegt ist, einen Luer-Steckverbinder reibschlüssig aufzunehmen, wobei die Nadel (5) zumindest an ihrem abschließenden Teil, das die Öffnung oder Öffnungen (8) umfasst, im Formhohlraum (17) einer elastischen Dichtung (13) eng eingeschlossen ist, die in der Dicke ihres freien Endes einen Schlitz (27) oder dergleichen aufweist, wobei die elastische Dichtung (13) ein freies Ende hat, das dasjenige der Kammer (2) tangiert und mit einem Ring (12) ausgestattet ist, der ihr abschließendes Teil zumindest bis in den Bereich gegenüber der oder den Öffnung/en (8) der Nadel einfasst, **dadurch gekennzeichnet, dass** über einen Teil der Länge des abschließenden Endes der Kammer (2) die Außenfläche der elastischen Dichtung (13) und/oder die Innenfläche oder die Dicke des Rings (12) mindestens eine Ausnehmung umfasst, die dazu bestimmt ist, das Zurückschieben des Materials, aus dem die Dichtung (13) besteht, zum Zeitpunkt des Durchtritts der Nadel (5) zu begünstigen.

2. Verbinder nach Anspruch 1, **dadurch gekennzeichnet, dass** die Ausnehmung an der Außenfläche det elastischen Dichtung (13) und in der Dicke des Rings (12) vorgesehen ist.

3. Verbinder nach einem der vorhergehenden Ansprüche, **dadurch gekennzeichnet, dass** die Außenfläche der elastischen Dichtung eine ringförmige Ausnehmung (14) aufweist, während der Ring in seiner Dicke zwei symmetrisch verteilte Ausnehmungen (15, 20) aufweist.

4. Verbindet nach Anspruch 1, **dadurch gekennzeichnet, dass** die Ausnehmung ausschließlich an der Außenfläche der elastischen Dichtung (13) vorgesehen ist.

5. Verbinder nach einem der Ansprüche 1 bis 4, **dadurch gekennzeichnet, dass** die Nadel (5) ein konisches, planes oder abgerundetes Ende aufweist und mit einer abschließenden CSffnung ausgestattet ist.

6. Verbinder nach Anspruch 5, **dadurch gekennzeichnet, dass** der äußere Abschnitt der Dichtung (13), der mit dem Ring (12) der Ausnehmung nachgeordnet in Kontakt ist, größer ist als der entsprechende innere Abschnitt des Rings (12).

7. Verbinder nach einem der Ansprüche 5 oder 6, **dadurch gekennzeichnet, dass** der oder den Ausnehmung/en (14, 15, 20) vorgeordnet, der äußere Abschnitt der Dichtung (13) im Wesentlichen gleich dem inneren Abschnitt des Rings (12) ist.

8. Verbinder nach einem der Ansprüche 1 bis 4, **dadurch gekennzeichnet, dass** die Nadel (5) ein konisches, planes oder abgerundetes Ende aufweist und mit zwei, einander gegenüberliegenden seitlichen Öffnungen (8) ausgestattet ist.

9. Verbinder nach Anspruch 8, **dadurch gekennzeichnet, dass** über die gesamte Länge des Rings (12) der äußere Abschnitt der Dichtung (13) mit Ausnahme der Ausnehmung im Wesentlichen gleich dem inneren Abschnitt: des Rings (12) ist.

10. Verbinder nach einem der Ansprüche 5 bis 9, **dadurch gekennzeichnet, dass** die Ausnehmung/en (14, 15, 20) in einem dem Ende der Nadel vorgeordneten Bereich ausgebildet ist/sind.

11. Verbinder nach einem der Ansprüche 5 bis 10, **dadurch gekennzeichnet, dass**:
- die Nadel (5) über den gesamten vom Ring (19) eingefassten Teil der elastischen Dichtung einen Abschnitt hat, der größer ist als der entsprechende innere Abschnitt der Dichtung (13),
- die Nadel (5) diesem Teil vorgeordnet einen Abschnitt hat, der im Wesentlichen gleich oder kleiner ist als der entsprechende innere Abschnitt der Dichtung (13).

12. Verbinder nach einem der vorhergehenden Ansprüche, **dadurch gekennzeichnet, dass** die elastische Dichtung (13) die gesamte Länge der Nadel (5) abdeckt und zwei seitliche Schlitze (16) aufweist, die in ihrem mittleren Teil vorgesehen sind.

13. Verbinder nach einem der Ansprüche 1 bis 11, **dadurch gekennzeichnet, dass** die elastische Dichtung (13) die gesamte Länge der Nadel (5) abdeckt und eine konische Form hat, und dass die Wand im Bereich ihrer mittleren Teile und ihres vorgeordneten Endes eine Abfolge von Verdickungen aufweist.

14. Verbinder nach einem der vorhergehenden Ansprüche, **dadurch gekennzeichnet, dass** die elastische Dichtung (13) in der nicht komprimierten Stellung keinen Kontaktpunkt mit den Seitenwänden der Kammer (2) hat.

15. Verbinder nach einem der vorhergehenden Ansprüche, **dadurch gekennzeichnet, dass** die gesamte Wand oder ein Teil der Wand des Rings (19)während der gesamten Dauer der Bewegung der elastischen Dichtung (12) von ihrer Ruhestellung in ihre komprimierte Stellung in Gleitkontakt mit der entsprechenden Wand der Kammer (2) ist.

16. Verbinder nach einem der vorhergehenden Ansprüche, **dadurch gekennzeichnet, dass** die Kammer (2) mindestens zwei Abteilungen mit unterschiedlichem Querschnitt aufweist, und zwar jeweils eine mittlere Abteilung (2b) mit zumindest in dem Bereich, der dem Weg des Rings(12) entspricht, konstantem innerem Querschnitt, und eine das abschließende Ende bildende abschließende Abteilung (2c) mit einem Querschnitt, der kleiner und konstant oder auch nicht ist, wobei sich der Ring (12) zum Teil in die mittlere Abteilung (2b) erstreckt.

17. Verbinder nach Anspruch 16, **dadurch gekennzeichnet, dass** der innere Abschnitt der abschließenden Abteilung einen 6 %-igen Luer-Konus aufweist, und dass der Teil des Rings, der in der abschließenden Abteilung enthalten ist, eine zylindrische Form hat.
